Europäisches Patentamt

European Patent Office

Office européen des brevets

(19)

(11) **EP 0 619 142 B1**

(12) **EUROPÄISCHE PATENTSCHRIFT**

(45) Veröffentlichungstag und Bekanntmachung des Hinweises auf die Patenterteilung:
**30.07.1997 Patentblatt 1997/31**

(51) Int. Cl.$^6$: **B01J 23/50**, C07D 301/10, B01J 23/66

(21) Anmeldenummer: **94101959.8**

(22) Anmeldetag: **09.02.1994**

(54) **Silberkatalysator zur Oxidation von Ethylen zu Ethylenoxid und Verfahren zur Herstellung von Ethylenoxid**

Silver catalyst for the oxidation of ethylene and process for the production of ethylene oxide

Catalyseur à base d'argent pour l'oxydation de l'éthylène et procédé de production de l'oxyde d'éthylène

(84) Benannte Vertragsstaaten:
**BE DE ES FR GB IT NL SE**

(30) Priorität: **08.04.1993 DE 4311608**

(43) Veröffentlichungstag der Anmeldung:
**12.10.1994 Patentblatt 1994/41**

(73) Patentinhaber: **HÜLS AKTIENGESELLSCHAFT**
**45764 Marl (DE)**

(72) Erfinder:
• **Burkhard, Klopries, Dr.**
  **D-46244 Bottrop (DE)**
• **Metz, Harald, Dr.**
  **D-45768 Marl (DE)**
• **Dibowski, Wilma**
  **D-45770 Marl (DE)**
• **Kyewski, Dietmar**
  **D-45721 Haltern (DE)**
• **Pospiech, Jürgen**
  **D-45768 Marl (DE)**

(56) Entgegenhaltungen:
**EP-A- 0 091 165**    **EP-A- 0 229 465**
**EP-A- 0 428 845**    **FR-A- 2 244 566**
**FR-A- 2 495 958**

**Beschreibung**

Silberkatalysatoren entsprechend dem Oberbegriff von Patentanspruch 1 sind seit langem bekannt (siehe beispielsweise EP-OS 0 172 565 und EP-OS 0 428 845). Die Silberkatalysatoren des Standes der Technik zeichnen sich bereits durch eine hohe Selektivität (bei hoher Aktivität) und ein günstiges Alterungsverhalten aus, welche die Wirtschaftlichkeit des Verfahrens zur Herstellung von Ethylenoxid bestimmen. In Anbetracht des verschärften Wettbewerbs zwischen den Ethylenoxidherstellern besteht ein Bedarf nach einem verbesserten Silberkatalysator mit erhöhter Selektivität (bei hoher Aktivität) und/oder günstigerem Alterungsverhalten und damit verbesserter Wirtschaftlichkeit eines Verfahrens zur Herstellung von Ethylenoxid.

Die Aufgabe der Erfindung besteht somit darin, einen Silberkatalysator bereitzustellen, der die Silberkatalysatoren des Standes der Technik hinsichtlich Selektivität (bei hoher Aktivität) und/oder Alterungsverhalten und damit hinsichtlich der Wirtschaftlichkeit des Verfahrens zur Herstellung von Ethylenoxid übertrifft.

Die Aufgabe wurde überraschenderweise wie in den Patentansprüchen 1 bis 7 angegeben gelöst.

Der Gegenstand der Erfindung besteht somit in einem Silberkatalysator mit einem Silbergehalt von 10 bis 25, vorzugsweise 15 bis 22 Massen-%, bezogen auf den fertigen Katalysator, zur partiellen Oxidation von Ethylen mit molekularem Sauerstoff in der Gasphase zu Ethylenoxid, wobei das Silber in Partikeln vorliegt, die sich auf einem porösen, nicht aziden, hitzebeständigen, keramischen Träger befinden und einen durch Rasterelektronenmikroskopie (REM) bestimmten, zahlenmittleren Durchmesser von 0,05 bis < 0,4, vorzugsweise 0,05 bis < 0,3 µm aufweisen. Bei der Bestimmung geht man so vor, daß man zunächst den fertigen Silberkatalysator, der in der Regel ringförmig vorliegt, durchbricht. Dann wird der gesamte Querschnitt geprüft. Der Gegenstand der Erfindung besteht außerdem in einem Verfahren zur partiellen Oxidation von Ethylen mit molekularem Sauerstoff in der Gasphase zu Ethylenoxid mit dem Silberkatalysator. Der Silberkatalysator ist durch die folgenden zusätzlichen Merkmale charakterisiert.

Der Träger ist makroporös. Unter Makroporen werden nach IUPAC Poren mit einem Durchmesser von > 50 nm verstanden. Poren mit einem Durchmesser zwischen 2 nm und 50 nm werden als Mesoporen bezeichnet. Die Makroporosität des Trägers wird durch ein durch Hg-Porosimetrie bei bis 2 000 bar ansteigendem Druck bestimmtes Porenvolumen von > 0,35, vorzugsweise > 0,40 cm$^3$/g des unbehandelten Trägers, also durch die untere Grenze des Porenvolumens, charakterisiert. Dabei werden die Porenvolumina der Makroporen und zum Teil auch der Mesoporen erfaßt (E. Robens, "The measurement of specific surface area and pore size distribution of powders", in Powder Metallurgy International, Vol. 18/1 (1986), Seiten 12 bis 16). Die Makroporosität des Trägers wird hinsichtlich der oberen Grenze des Porenvolumens durch die Bedingung charakterisiert, daß die Seitenbruchfestigkeit des fertigen Katalysators 20 N nicht unterschreitet (E. R. Beaver, Mechanical Testing of Extruded, Tableted and Ring-formed Catalysts, American Institute of Chemical Engineers, Symposium Series, Vol. 143/70 (1974), Seiten 1 bis 4). Überraschenderweise ist die Seitenbruchfestigkeit des fertigen Katalysators deutlich erhöht gegenüber derjenigen des Trägers.

Der mittlere Durchmesser der Silberpartikel soll der zusätzlichen Bedingung genügen, daß er kleiner ist als das 0,4fache, vorzugsweise kleiner ist als das 0,3fache des zahlenmittleren Porendurchmessers des unbehandelten Trägers. Der zahlenmittlere Porendurchmesser wird aus der durch Hg-Porosimetrie bei bis 2 000 bar ansteigendem Druck bestimmten Porenradienverteilung abgeleitet. Dabei werden die Makroporen und zum Teil auch die Mesoporen erfaßt. Der unbehandelte Träger weist einen mittleren Porendurchmesser von ≥ 1 µm auf.

Die Silberpartikel in der äußeren Oberfläche des fertigen Katalysators sollen im wesentlichen keine Silberbrücken bilden, so daß der fertige Katalysator sich wie ein Isolator mit hohem elektrischen Widerstand verhält. Zur Prüfung ist ein hochohmiges Widerstandsmeßgerät (Meßbereich 0 bis 200 MΩ) mit zwei Punktelektroden im Abstand von 1 mm geeignet. Man tastet die Oberfläche der Katalysatorteilchen mit den Elektroden ab. Der Silberkatalysator besteht die Prüfung nicht, wenn das Widerstandsmeßgerät einen Wert im angegebenen Meßbereich anzeigt. Der Silberkatalysator der EP-OS 0 428 845 beispielsweise besteht die Prüfung nicht. Ein geeigneter Silberkatalysator hat demnach bei der beschriebenen Meßanordnung einen elektrischen Widerstand, der 200 MΩ überschreitet.

Die Silberpartikel haben in der Regel eine kugelähnliche Gestalt. Sie sind in REM-Aufnahmen bei 50 000facher Vergrößerung deutlich zu erkennen. Unvorteilhaft ist eine nahezu punktförmige Auflage der Silberpartikel auf der Oberfläche des Trägers, die ihrerseits strukturiert ist. Legt man eine Tangente an die Abbildung des Silberpartikels in Richtung auf die Grenze von Silberpartikel und Oberfläche des Trägers, so kann man einen Winkel zwischen Tangente und Oberfläche des Trägers messen. Dieser Winkel ist als Randwinkel definiert. Im Falle einer nahezu punktförmigen Auflage ist er < 30 °. Ein kleiner Randwinkel wirkt sich ungünstig auf das Alterungsverhalten des Silberkatalysators aus. Vorzugsweise beträgt der Randwinkel im Mittel > 30, insbesondere > 50 °. Bei der Prüfung geht man so vor, daß man zunächst den fertigen Silberkatalysator durchbricht. Dann wird der gesamte Querschnitt geprüft. Überraschenderweise läßt sich ein großer Randwinkel durch Dotierung des metallischen Silbers mit einer kationischen Komponente aus der Gruppe der Verbindungen von Li, Na, K, Cs, Mg, Ca, Ba, Ti, V, Cr und Re einstellen. Bevorzugt ist ein Silberkatalysator, in dem der Randwinkel durch Dotierung des Silbers mit einer Cäsiumverbindung eingestellt ist.

Ein bevorzugter Träger weist eine durch Hg-Porosimetrie bei bis 2 000 bar ansteigendem Druck bestimmte Oberfläche der Makroporen von > 0,8 m$^2$/g des unbehandelten Trägers auf.

Im Hinblick auf eine hohe Selektivität des Silberkatalysators weist der Katalysatorträger möglichst wenig Mikroporen und Mesoporen auf. Unter Mikroporen werden nach IUPAC Poren mit einem Durchmesser zwischen 0,6 nm und 2 nm verstanden. Das Porenvolumen des Katalysatorträgers im Mikro- und Mesoporenbereich wird aus der Physisorptionsisotherme von Stickstoff bei der Temperatur des flüssigen Stickstoffs bestimmt. Die Messung wird in Anlehnung an die DIN 66 131 (BET-Methode) durchgeführt. Die Auswertung erfolgt gemäß dem Verfahren von Barret, Joyner und Halenda aus der Desorptionsisotherme (J. Am. Chem. Soc. 73 (1951), Seite 373) . Im Hinblick auf eine hohe Selektivität des Silberkatalysators soll das BET-bestimmte Porenvolumen des Trägers möglichst gering sein. Ein bevorzugter Träger weist ein BET-bestimmtes Porenvolumen von < 0,03, insbesondere < 0,02 cm$^3$/g des unbehandelten Trägers auf.

Ein bevorzugter Träger besteht zu > 80, besonders bevorzugt zu > 90 Massen-% aus $Al_2O_3$, insbesondere $\alpha$-$Al_2O_3$. Die Silberpartikel sollen gleichmäßig auf der für Gase frei zugänglichen Oberfläche des Trägers verteilt sein. Die Gleichmäßigkeit der Verteilung der Silberpartikel auf der für Gase frei zugänglichen Oberfläche des Trägers ist durch den mit der ESCA-Methode bestimmten, mittleren Belegungsgrad der für Gase frei zugänglichen Oberfläche des Trägers mit Silber charakterisiert. Der Belegungsgrad ist durch den Quotienten

$$\frac{Ag\ (Atom\text{-}\%)}{Ag\ Atom\text{-}\% + Al\ (Atom\text{-}\%)}$$

definiert. Bei der Prüfung geht man so vor, daß man zunächst den fertigen Silberkatalysator durchbricht. Man bestrahlt die Bruchflächen mehrerer Katalysatorteilchen mit Röntgenstrahlen und zwar Kreisflächen mit dem Durchmesser 0,33 mm a) in der Mitte der Bruchfläche ("im Innenbereich") und b) im Abstand von 0,3 mm von den äußeren Rändern der Bruchflächen ("im Randbereich"). Die aus der Oberfläche herausgeschlagenen Elektronen weisen je nach Art der Elemente unterschiedliche Energien auf. Die Intensität der ausgelösten Elektronenstrahlung ist ein Maß für den Mengenanteil des Elements in der Oberfläche (S. Svanberg, Atomic and Molecular Spectroscopy, Basic Aspects und Practical Applications, Springer Verlag, 1991). Der Belegungsgrad soll sowohl bei der Meßbedingung a) als auch bei der Meßbedingung b) jeweils im Mittel > 0,3, insbesondere > 0,4 betragen.

Der Silberkatalysator wird nach grundsätzlich bekannten Methoden hergestellt, d. h. im allgemeinen durch die folgenden Verfahrensschritte: Tränken (Imprägnieren) des Trägers bei vermindertem Druck mit einer wäßrigen (kolloidalen) Lösung eines Silbersalzes, deren Oberflächenspannung vorteilhafterweise durch ein geeignetes Tensid auf < 50 mN/m eingestellt ist, Trocknung und Pyrolyse. Durch die letzteren Verfahrensschritte wird das metallische Silber auf dem Träger abgeschieden. Geeignet ist beispielsweise eine Silberlactat- oder Silberoxalat-Aminkomplexlösung, deren Oberflächenspannung mit einem Laurylaminethoxylat (10 Mol Ethylenoxid/Mol Laurylamin) eingestellt ist. Geeignete, komplexierende Amine sind beispielsweise Monoethanolamin und Ethylendiamin. Nach der Trocknung können sich eine zweite Tränkung und eine zweite Trocknung anschließen. Die Trocknung erfolgt im allgemeinen bei 105 bis 150 °C. Die Pyrolyse erfolgt durch Erhitzen mit einer Aufheizrate von $\geq$ 1 °C/s auf $\geq$ 180 °C. Sie wird durch die sogenannte Endformierung bei $\leq$ 240 °C beendet.

Gemäß der Lehre der Erfindung ist es möglich, Silberkatalysatoren mit einer großen Silberoberfläche herzustellen. Sie haben dementsprechend eine hohe Aktivität. Diese erlaubt in einem Verfahren zur Herstellung von Ethylenoxid mit dem Silberkatalysator die Einstellung einer vergleichsweise niedrigen Reaktionstemperatur und dementsprechend die Einstellung eines vergleichsweise hohen Sauerstoffgehaltes im rückgeführten Kreisgas. Die niedrige Reaktionstemperatur, die im allgemeinen 235, vorzugsweise 225 °C nicht überschreitet, wirkt sich günstig auf das Alterungsverhalten aus.

Die Erfindung wird durch das folgende Beispiel erläutert. Darin bedeutet Prozent (%) Massen-%.

Beispiel 1 (Herstellung des Silberkatalysators)

Eingesetzt wurde ein ringförmiger Katalysatorträger, der wie folgt charakterisiert ist:

| | |
|---|---|
| äußerer Teilchendurchmesser/innerer T./Höhe [mm] | 6,5/3/6 |
| Schüttgewicht [g/l] | 747 |
| Wasseraufnahmevermögen, bezogen auf den trocknen Träger [%] | ca. 50 |
| Seitenbruchfestigkeit [N] | 33 |
| $Al_2O_3$-Gehalt [%]* | > 97 |
| Volumen der Makroporen ($cm^3/g$]** | 0,47 |
| Oberfläche der Makroporen [$m^2/g$]** | 1,03 |
| zahlenmittlerer Durchmesser der Makroporen [$\mu$m]** | 4,1 |
| Volumen der Mikroporen und Mesoporen [$cm^3/g$]*** | < 0,01 |

\* vorliegend als $\alpha$-$Al_2O_3$
\*\* bestimmt durch Hg-Porosimetrie mit dem Porosimeter 2 000 der Fa. Carlo Erba
\*\*\* bestimmt durch die BET-Methode

Zu einer Lösung von 1 778 g Silbernitrat in 1,7 l vollentsalztem Wasser wurden 1,6 l einer 25 %igen, wäßrigen Natronlauge gegeben. Das ausgefällte Silberhydroxid wurde filtriert, mit vollentsalztem Wasser nitratfrei gewaschen und mit vollentsalztem Wasser zu einem Brei gerührt. Unter Kühlung wurden 692 g kristalline Oxalsäure eingetragen. Das erhaltene Silberoxalat wurde mit 566 g Ethylendiamin, 296 g Monoethanolamin und 4,0 g Laurylaminethoxylat (10 Mol Ethylenoxid/Mol Laurylamin) versetzt. In die so erhaltene, konzentrierte Silberoxalataminkomplexlösung wurden 2 436 g des oben beschriebenen, trockenen Trägers eingetragen. Das Gemisch wurde durch Rotation in einem geeigneten Gefäß bewegt und auf ca. 40 °C erhitzt. Zur Entgasung des getränkten Trägers wurde der Druck bis zum leichten Sieden vermindert. Anschließend wurde die überstehende Flüssigkeit dekantiert und der behandelte Träger bei 105 bis 120 °C im Stickstoffstrom getrocknet. Der trockene Träger wurde mit einem Gemisch aus der dekantierten Flüssigkeit und einer wäßrigen Lösung von 2,84 g $C_sNO_3$ versetzt. Wieder wurde der getränkte Träger wie oben bei ca. 40 °C unter vermindertem Druck entgast. Sodann wurde der behandelte Träger bei 105 bis 150 °C im Stickstoffstrom getrocknet. Zur Pyrolyse wurde der behandelte Träger mit einer Geschwindigkeit von ≥ 1 °C/s bis auf 210 °C aufgeheizt und bei dieser Temperatur 1 Std. erhitzt. Dabei wurde er mit einem Stickstoff/Wasserstoff-Strom mit einem Anteil von 4 Vol.-% Wasserstoff gespült.

Der so erhaltene, fertige Silberkatalysator ist wie folgt charakterisiert:

| | | |
|---|---|---|
| Seitenbruckfestigkeit [N] | | 42 |
| elektrischer Widerstand [M$\Omega$] | | > 200 |
| Silbergehalt [%] | | 20 |
| mittlerer Belegungsgrad | im Randbereich | 0,47 |
| | im Innenbereich | 0,46 |
| Randwinkel [°] | | 50 bis 60 |
| zahlenmittlerer Durchmesser der Silberpartikel [$\mu$m] | | 0,15 |

$$\frac{\text{mittlerer Durchmesser der Silberpartikel [}\mu\text{m]}}{\text{mittlerer Durchmesser der Makroporen des Trägers [}\mu\text{m]}} = \frac{0,15}{4,1} = 0,037$$

**Patentansprüche**

1. Silberkatalysator mit einem Silbergehalt von 10 bis 25 Massen-%, bezogen auf den fertigen Katalysator, zur partiellen Oxidation von Ethylen mit molekularem Sauerstoff in der Gasphase zu Ethylenoxid, wobei das Silber in Partikeln vorliegt, die sich auf einem porösen, nicht aziden, hitzebeständigen, keramischen Träger befinden und einen durch Rasterelektronenmikroskopie bestimmten, zahlenmittleren Partikeldurchmesser von 0,05 bis < 0,4 μm aufweisen,

dadurch gekennzeichnet, daß

- der Träger makroporös ist, wobei die Makroporosität durch ein durch Hg-Porosimetrie bei bis 2 000 bar ansteigendem Druck bestimmtes Porenvolumen mit einer unteren Grenze von > 0,35 cm$^3$/g des unbehandelten Trägers charakterisiert ist und hinsichtlich der oberen Grenze des Porenvolumens durch die Bedingung charakterisiert ist, daß die Seitenbruchfestigkeit des fertigen Katalysators 20 N nicht unterschreitet;
- der mittlere Durchmesser der Silberpartikel der zusätzlichen Bedingung genügt, daß er kleiner ist als das 0,4fache des zahlenmittleren Porendurchmessers des unbehandelten Trägers, wobei der zahlenmittlere Porendurchmesser aus der durch Hg-Porosimetrie bei bis 2 000 bar ansteigendem Druck bestimmten Porenradienverteilung abgeleitet ist und ≥ 1 μm beträgt;
- die Silberpartikel in der äußeren Oberfläche des fertigen Katalysators im wesentlichen keine Silberbrücken bilden, so daß der fertige Katalysator sich wie ein Isolator mit hohem elektrischen Widerstand verhält.

2. Silberkatalysator nach Anspruch 1,
   dadurch gekennzeichnet,
   daß der Randwinkel zwischen Silberpartikel und Träger im Mittel > 30, insbesondere > 50 ° beträgt.

3. Silberkatalysator nach Anspruch 2,
   dadurch gekennzeichnet,
   daß der Randwinkel durch Dotierung des metallischen Silbers mit einer kationischen Komponente aus der Gruppe der Verbindungen von Li, Na, K, Cs, Mg, Ca, Ba, Ti, V, Cr und Re, insbesondere mit einer Cäsiumverbindung eingestellt ist.

4. Silberkatalysator nach einem der Ansprüche 1 bis 3,
   dadurch gekennzeichnet,
   daß der Träger eine durch Hg-Porosimetrie bei bis 2 000 bar ansteigendem Druck bestimmte Oberfläche der Makroporen von > 0,8 m$^2$/g des unbehandelten Trägers aufweist.

5. Silberkatalysator nach einem der Ansprüche 1 bis 4,
   dadurch gekennzeichnet,
   daß der Träger ein durch die BET-Methode bestimmtes Porenvolumen von < 0,03, insbesondere < 0,02 cm$^3$/g des unbehandelten Trägers aufweist.

6. Silberkatalysator nach einem der Ansprüche 1 bis 5,
   dadurch gekennzeichnet,
   daß der Träger zu > 80, vorzugsweise > 90 Massen-% aus Al$_2$O$_3$, insbesondere α-Al$_2$O$_3$ besteht.

7. Verfahren zur partiellen Oxidation von Ethylen mit molekularem Sauerstoff in der Gasphase zu Ethylenoxid mit einem Silberkatalysator nach einem der Ansprüche 1 bis 6.

## Claims

1. A silver catalyst having a silver content of from 10 to 25% by weight, based on the finished catalyst, for the partial oxidation of ethylene with molecular oxygen in the gas phase to form ethylene oxide, the silver being present in particles which are situated on a porous, nonacidic, heat resistant, ceramic support and have a number-average particle diameter from 0.05 to < 0.4 μm determined by scanning electron microscopy, characterized in that

   - the support is macroporous, where the macroporosity is characterized by a pore volume having a lower limit of > 0.35 cm$^3$/g of the untreated support, determined by Hg porosimetry at a pressure rising to 2000 bar, and with respect to the upper limit of the pore volume is characterized by the lateral fracture strength of the finished catalyst being not less than 20 N;
   - the average diameter of the silver particles satisfies the additional condition of being less than 0.4 times the number-average pore diameter of the untreated support, the number-average pore diameter being derived from the pore radius distribution determined by Hg porosimetry at a pressure rising to 2000 bar and being ≥ 1 μm;

- the silver particles in the outer surface of the finished catalyst form essentially no silver bridges, so that the finished catalyst behaves like an insulator with high electrical resistance.

2. A silver catalyst according to claim 1, characterized in that the contact angle between silver particles and support has an average value > 30°, in particular > 50°.

3. A silver catalyst according to claim 2, characterized in that the contact angle is adjusted by doping the metallic silver with a cationic component selected from the group of compounds of Li, Na, K, Cs, Mg, Ca, Ba, Ti, V, Cr and Re, in particular with a caesium compound.

4. A silver catalyst according to any one of claims 1 to 3, characterized in that the support has a surface area of the macropores of > 0.8 $m^2$/g of the untreated support, determined by Hg porosimetry at a pressure rising to 2000 bar.

5. A silver catalyst according to any one of claims 1 to 4, characterized in that the support has a BET pore volume of < 0.03, in particular < 0.02, $cm^3$/g of the untreated support.

6. A silver catalyst according to any one of claims 1 to 5, characterized in that the support consists to an extent of > 80% by weight, preferably > 90% by weight, of $Al_2O_3$, in particular $\alpha$-$Al_2O_3$.

7. A process for the partial oxidation of ethylene with molecular oxygen in the gas phase to form ethylene oxide using a silver catalyst according to any one of claims 1 to 6.

**Revendications**

1. Catalyseur à l'argent ayant une teneur en argent de 10 à 25 % en masse, rapporté au catalyseur terminé, en vue de l'oxydation partielle de l'éthylène avec de l'oxygène moléculaire en phase gazeuse, en oxyde d'éthylène, procédé dans lequel l'argent se présente en particules qui se trouvent sur un support poreux, non azide, stable au chauffage, céramique, et qui possèdent un diamètre de particules moyen numérique déterminé par microscopie d'électrons, de balayage de 0,05 à < 0,4 $\mu$m,
   caractérisé en ce que

   - le support est macroporeux pour lequel la macroporosité est caractérisée par un volume de pores avec une limite inférieure de < 0,35 $cm^3$/g du support non traité, déterminé par porosimétrie au Hg, à une pression croissante allant jusqu'à 2000 bars et en ce qui concerne la limite supérieure du volume des pores, est caractérisée par la condition que la résistance à la rupture latérale du catalyseur terminé ne dépasse pas 20 N,
   - le diamètre moyen des particules d'argent satisfait à la condition supplémentaire qu'il est plus petit que 0,4 fois le diamètre de pore moyen numérique du support non traité, pour lequel le diamètre moyen numérique des pores, dérive de la distribution des rayons de pores déterminée par porosimétrie au Hg, à une pression croissante jusqu'à 2000 bars et s'élève à ≥ 1 $\mu$m,
   - les particules d'argent dans la surface externe du catalyseur terminé, ne forment essentiellement aucun pont d'argent, de sorte que le catalyseur terminé se comporte comme un isolateur avec une résistance électrique élevée.

2. Catalyseur à l'argent selon la revendication 1,
   caractérisé en ce que
   l'angle de bordure entre les particules d'argent et le support s'élève en moyenne à > 30°, en particulier à > 50°.

3. Catalyseur à l'argent selon la revendication 2,
   caractérisé en ce que
   l'angle de bordure est ajusté par dopage de l'argent métallique avec un composant cationique choisi dans le groupe des composés du Li, Na, K, Cs, Mg, Ca, Ba, Ti, V, Cr et Re en particulier avec un dérivé du Césium.

4. Catalyseur à l'argent selon l'une des revendications 1 à 3,
   caractérisé en ce que
   le support possède une surface déterminée par porosimétrie au Hg à une pression croissante jusqu'à 2000 bars, des macropores de > 0,8 $m^2$/g du support non traité.

5. Catalyseur à l'argent selon l'une des revendications 1 à 4,
   caractérisé en ce que

le support possède un volume de pores déterminé par la méthode BET, de < 0,03, en particulier < 0,02 cm$^3$/g de support non traité.

6. Catalyseur à l'argent selon l'une des revendications 1 à 5,
caractérisé en ce que
le support consiste pour plus de 80, de préférence plus de 90 % en masse d'Al$_2$O$_3$, en particulier Al$_2$O$_3$-$\alpha$.

7. Procédé d'oxydation partielle de l'éthylène avec de l'oxygène moléculaire, en phase gazeuse, en oxyde d'éthylène, avec un catalyseur à l'argent selon l'une des revendications 1 à 6.